# EUROPEAN PATENT SPECIFICATION

(11) **EP 1 977 699 B1**
(45) Date of publication and mention of the grant of the patent: **30.12.2020**
(21) Application number: 08161256.6
(22) Date of filing: 05.05.2005
(51) Int. Cl.: A61F 2/00, A61B 17/04, A61B 17/06

(54) **Kit for repairing cystocele**
Set zum Reparieren der Cystocele
Nécessaire de réparation de la cystocele

(30) Priority: 07.05.2004 US 840646
(43) Date of publication of application: 08.10.2008
(62) Document number(s) of the earlier application(s) in accordance with Art. 76 EPC: 05741770.1 / 1 750 613
(73) Proprietor: AMS Research Corporation, Minnetonka, MN 55343 (US)
(72) Inventor: Montpetit, Karen Pilney, Minnetonka, MN 55343 (US); Anderson, Kimberly A., Minnetonka, MN 55343 (US); Watschke, Brian P., Minnetonka, MN 55343 (US)
(74) Representative: Vossius & Partner Patentanwälte Rechtsanwälte mbB

(56) References cited:
- WO-A-03/073960
- WO-A-2004/091443
- US-A1- 2004 039 453

## Description

### Urogenital Surgery

Female genital prolapse has long plagued women. It is estimated by the U.S. National Center for Health Statistics that 247,000 operations for genital prolapse were performed in 1998. With the increasing age of the U.S. population, these problems will likely assume additional importance.

Vaginal prolapse develops when intra-abdominal pressure pushes the vagina outside the body. In a normal situation, the levator ani muscles close the pelvic floor. This results in little force being applied to the fasciae and ligaments that support the genital organs. Increases in abdominal pressure, failure of the muscles to keep the pelvic floor closed, and damage to the ligaments and fasciae all contribute to the development of prolapse.

Many techniques have been tried to correct or ameliorate the prolapse and its symptoms, with varying degrees of success. Nonsurgical treatment of prolapse involves measures to improve the factors associated with prolapse, including treating chronic cough, obesity, and constipation. Other nonsurgical treatments may include pelvic muscles exercises or supplementation with estrogen. These therapies may alleviate symptoms and prevent worsening, but the actual hernia will remain. Vaginal pessaries are the primary type of nonsurgical treatment, but there can be complications due to vaginal wall ulceration.

There is a desire to obtain a minimally invasive yet highly effective device and method that can be used to treat pelvic organ prolapse with minimal to no side effects. Such a device should reduce the complexity of the surgical procedure, be biocompatible, adjustable, and non-toxic. The treatment methods using the device should reduce pain, operative risks, infections and post operative hospital stays. Further, the method of treatment should also improve the quality of life for patients.
WO 2004/091443 is directed towards an implant for the treatment of cystocele. The implant comprises a support body and six suspension harnesses.
US 2004/0039453 is directed towards an implant for treating pelvic disorders. The implant comprises a major portion and two sling appendages.
WO 2003/073960 is directed to an implant for treating central and lateral cystoceles. The implant comprises an anterior portion and laterally extending stabilizing straps.

The present invention provides a kit for cystocele repair according to claim 1 and the dependent claims.

A more complete appreciation of the invention and many of the attendant advantages thereof will be readily obtained as the same becomes better understood by reference to the following detailed description when considered in connection with the accompanying drawings, wherein:
Figure 1 is a perspective view of a support apparatus used in the present invention;
Figure 2 is a fragmentary front view of a first example of the support apparatus;
Figure 3 is a side view of a strap of a support apparatus;
Figure 4 is a top view of a front view of a strap of a support apparatus;
Figure 5 is a fragmentary front view of a second example of a support apparatus. used in the present invention;
Figure 6 is a fragmentary front view of a third example of a support apparatus used in the present invention;
Figure 7 is a front view of a biologic graft attached to a central portion of two straps, each strap having a connector at each end;
Figure 8 is a front view of a support member including both a biologic graft and a synthetic support member;
Figure 8A is a side view of a support member including both a biologic graft and a synthetic support member;
Figure 9 is a front view of a support member of the second example of the support apparatus;
Figure 10 is a front view of a support member of the first example of the support apparatus;
Figure 11 is a close up view of the weave pattern of an example of the support apparatus;
Figure 11A is a close up view of an alternate weave pattern for the support member;
Figure 12 is a front view of a surgical kit of an embodiment of the present invention;
Figure 13 is a perspective view of an embodiment of a right superior needle (the superior needle held in the surgeon's right hand) of the present invention;
Figure 14 is a top view of an embodiment of the right superior needle of the present invention;
Figure 15 is a bottom view of an embodiment of the right superior needle of the present invention;
Figure 16 is a left side view of an embodiment of the right superior needle of the present invention;
Figure 17 is a right side view of an embodiment of the right superior needle of the present invention;
Figure 18 is a front view of an embodiment of the right superior needle of the present invention;
Figure 19 is a rear view of an embodiment of the right superior needle of the present invention;
Figure 20 is a side perspective view of an embodiment of a left inferior needle shaft of the present invention, without a handle;
Figure 21 is a front perspective view of an embodiment of the left inferior needle shaft of the present invention, without a handle;
Figure 22 is a right side view of an embodiment of a left inferior needle shaft of the present invention, without a handle;
Figure 23 is a bottom view of an embodiment of the left inferior needle shaft of the present invention, without a handle;
Figure 24 is a front view of an embodiment of the left inferior needle shaft of the present invention, without a handle;
Figure 25 is a front view of an embodiment of a left superior strap connector having symbolic indicia thereon;
Figure 26 is a front view of an embodiment of a right superior strap connector having symbolic indicia thereon;
Figure 27 is a front view of an embodiment of a left inferior strap connector having symbolic indicia thereon;
Figure 28 is a front view of an embodiment of a right inferior strap connector having symbolic indicia thereon;
Figure 29 is a front view of an embodiment of a set of four needles and a support apparatus with four connectors, wherein the connectors are matched to the needles using colors;
Figure 30 is a perspective view of a first needle tip and connector of an embodiment of the present invention;
Figure 31 is a perspective view of a second needle tip and connector of an embodiment of the present invention;
Figure 32 is a front view of a patient showing the four needle entry incisions;
Figure 33 is a perspective view of a right superior needle tip entering the left superior incision (the superior incision on the patient's left side);
Figure 34 is a perspective view of a right superior needle tip exiting the vaginal incision;
Figure 35 is a front view of a right superior needle tip exiting the vaginal incision;
Figure 36 is a perspective view of a right superior needle tip connected to the right superior connector (the superior connector on the surgeon's right side);
Figure 37 is a perspective view of the superior straps and the support member in place and the inferior straps extending outside the vaginal incision;
Figure 38 is a perspective view of a right inferior needle tip exiting the vaginal incision;
Figure 39 is a perspective view of all the straps and the support member in place and the sheaths removed;
Figure 40 is a flow chart illustrating a method of using the claimed kit not forming part of the invention and
Figure 41 is a flow chart illustrating an alternate method of using the claimed kit, not forming part of the invention.

### DETAILED DESCRIPTION OF THE PREFERRED EMBODIMENTS

Referring now to the drawings, wherein like reference numerals designate identical or corresponding parts throughout the several views.

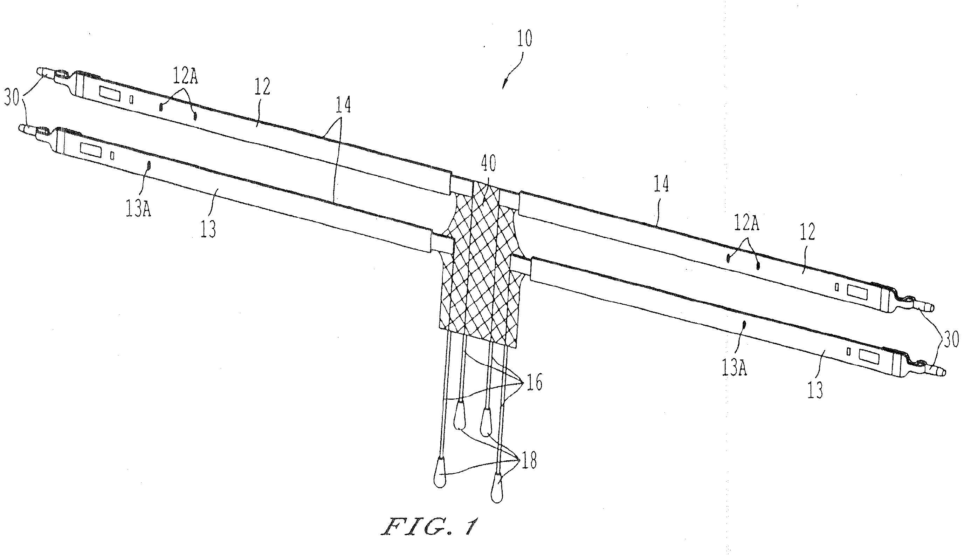

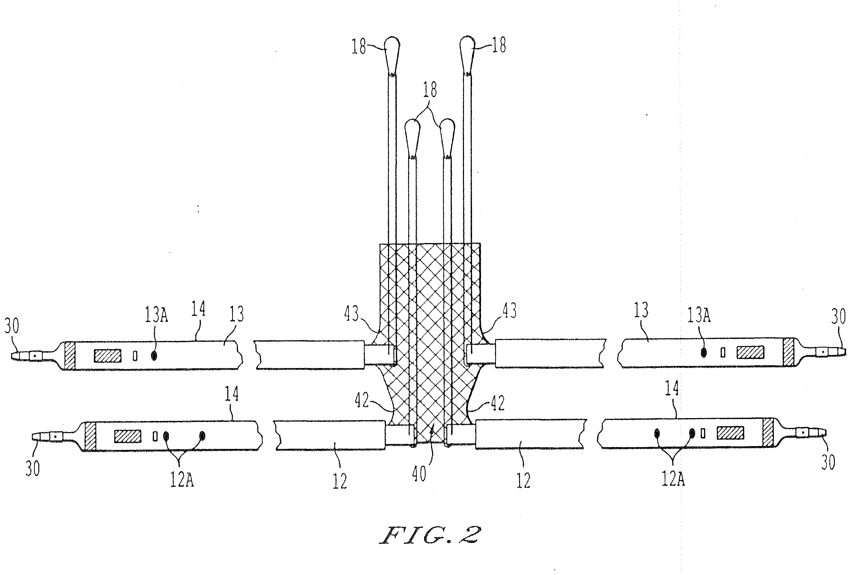

Figures 1 and 2 illustrate a surgical support apparatus 10 of a first example forming part of the kit according to the present invention. The apparatus 10 is configured to be surgically implanted in a female patient to repair anterior prolapse of the vagina. The present invention may be used to correct central defects, midline defects, or both midline and central defects at once. In the embodiment shown in Figures 1 and 2, apparatus 10 comprises two superior straps 12, two inferior straps 13, a support member 40, and four loosening sutures 16. Each of straps 12 and 13 include a connector 30. Each strap 12 and 13 is covered by a sheath 14. Each suture 16 includes a tab 18. Straps 12 and 13 are connected to tabs 42 and 43 of support member 40 by known means.

In one embodiment, sheath 14 is made of polyethylene. Other material may be used, such as polypropylene, nylon, polyester, or Teflon. The sheath is configured to be removed from the strap after the strap is in the correct position in the body.

In one embodiment, straps 12 and 13 are 50.0 cm (19.96 inches) long and 1.1 cm (0.433 inches) wide. The straps are 0.060 cm (0.024 inches) thick. Straps 12 and 13 are knitted of 0.1 or 0.15 mm (4 or 6 mil) polypropylene monofilament and are heat set at 138-149°C (280-200 degrees Fahrenheit) for 5-8 minutes. Also, in one embodiment, support member 40 is 10 cm long by 5 cm wide and 0.053 cm (0.021 inches) thick. Member 40 is knitted of 0.1 mm (4mil) polypropylene monofilament and heat set at 154-166°C (310-330 degrees Fahrenheit) for 5-8 minutes. Both the strap and support member have a stitch count of 27.5 courses/2.54 cm (inch)(±2 courses) and 13 wales/2.54 cm (inch)(±2 wales).

In one embodiment, the straps are knitted with bar settings of: Bar 1: 1/0, 2/1 and Bar 2: 0/1, 1/2. The support member is a large pore mesh, knitted with bar settings of: Bar 1: 1/0, 2/3, 2/1, 2/3, 1/0, 1/2, 1/0, 1/2; Bar 2: 1/0, 2/3, 2/3, 1/0; and Bar 3: 2/3, 1/0, 1/2, 1/0, 2/3, 2/1, 2/3, 2/1. The straps are connected to the support member after knitting. Weaving according to a given bar pattern is described, for example, in "Warp Knitting Production" by Dr. S. Raz, Melliand Textilberichte GmbH, Rohrbacher Str. 76, D-6900 Heidelberg, Germany (1987).

Straps 12 and 13 and or sheaths 14 may also include indicia thereon to signify the correct orientation for implantation into a patient. In the embodiment shown in Figures 1 and 2, sheaths 14 around straps 12 include indicia 12A to show that straps 12 are the superior straps, and sheaths 14 around straps 13 include indicia 13A to show that straps 13 are the inferior straps. Words, symbols, and colors are all possible indicia that may be used, and these modifications are intended to be within the scope of the invention as claimed. Further, the indicia may be located on the straps, the sheaths, or both.

Apparatus 10 includes dilating connectors 30. Suitable dilating connectors are disclosed in Published U.S. Patent Application Serial Nos. 2002/151762 and 2002/147382 and U.S. Patent Application Serial No. 10/386,897, filed Mar. 11, 2003.

Support member 40 is sized and shaped to afford repair of a cystocele without lifting the patient's bladder and without placing undue tension on the bladder or vaginal wall. The shape of member 40 may be predetermined, or the member may be trimmed based on patient anatomy before implantation.

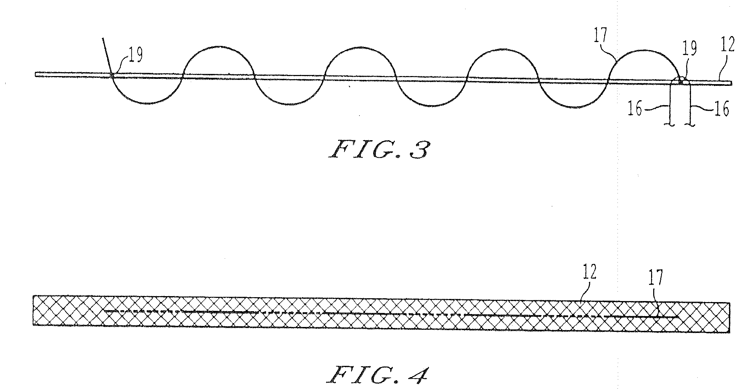

Figures 3 and 4 illustrate an embodiment of a strap for a surgical apparatus used in the present invention. In one embodiment, strap 12 includes tensioning suture 17. Tensioning suture 17 passes through the mesh of strap 12 multiple times, as shown in Figures 3 and 4. Tensioning suture 17 is affixed to strap 12 at points 19, to allow transfer of tension from the suture to the strap. In one embodiment, tensioning sutures are included in all the straps of the support apparatus. It should be readily apparent to one skilled in the art that other configurations of tensioning sutures and attachment points to a mesh strap are possible, and these modifications are within the scope of the invention as claimed.
6

Tensioning suture 17 is configured to eliminate slack in a strap that is already surgically implanted in the body. By tightening the strap with suture 17, rather than pulling on the strap itself, the surgeon prevents damage to the strap due to deformation. Damage to surrounding tissues due to excessive movement of the strap during adjustment is also avoided. Straps including tensioning sutures are disclosed in United States Patent No. 7,083,568.

Strap 12 also includes a connection point for loosening suture 16. As discussed below, loosening suture 16 is pulled by the surgeon to loosen the installed support member, if necessary.

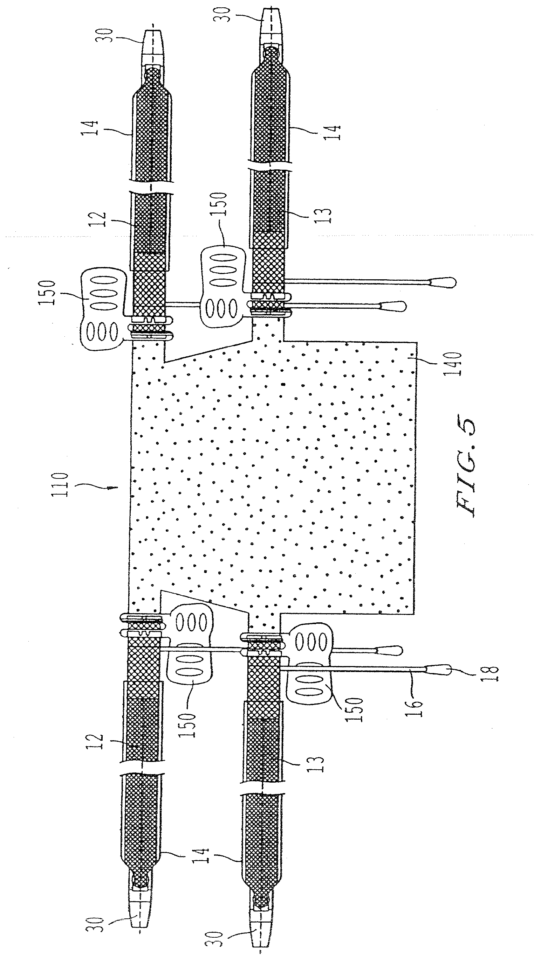

Figure 5 illustrates a second example of the surgical support being part of the kit according to the present invention. Apparatus 110 includes a biological graft for a support member 140. To attach the graft to straps 12 and 13, clamps 150 are used to hold the surfaces of the strap and member together. The surfaces are then secured together, as discussed below in the method for preparing the biologic graft. Straps 12 and 13, sheaths 14, and connectors 30 are described above.

In another embodiment, biologic graft comes in a kit already secured to straps 12 and 13. In this case, the preparation method below is unnecessary.

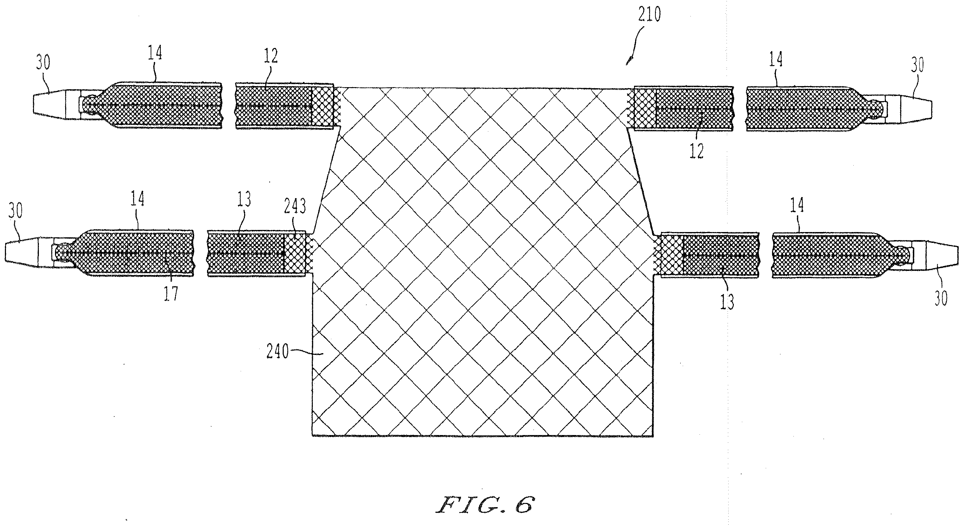

Figure 6 shows a third example of the surgical support apparatus being part of the kit used in the present invention. Apparatus 210 includes straps 12 and 13, sheaths 14, and support member 240. In this embodiment, support member 240 and straps 12 and 13 are continuously knitted. Thus, there is no seam between the straps and support member, as they are one continuous piece. This results in a thinner transition area 243 from the straps to the support member, which results in a less bulky apparatus for installment into the patient. An apparatus that is less bulky will be less likely to abrade the surrounding tissue.

The support member is knitted with a first bar pattern, and the straps are knitted with a second bar pattern. This allows larger pores in the support member, creating a support member that is more flexible and more likely to allow tissue ingrowth. A second bar pattern for the straps allows a smaller pore size for the straps, creating a strap that can carry a larger load with a smaller, less intrusive strap width.

In one embodiment, the straps and support member are continuously knitted of 0.1 mm (4mil) polypropylene monofilaments, knitted with a warp tricot. The stitch count is 27.5 courses/2.54 cm (inch)(±2 courses) and 13 wales/2.54 cm (inch)(±2 wales). The support member is a large pore mesh with bar settings of: Bar 1: 1/0, 2/3, 2/1, 2/3, 1/0, 1/2, 1/0, 1/2; ; Bar 2: 1/0, 2/3, 2/3, 1/0; and Bar 3: 2/3, 1/0, 1/2, 1/0, 2/3, 2/1, 2/3, 2/1. The thickness of the support member is about 0.53 cm (0.21 inches).

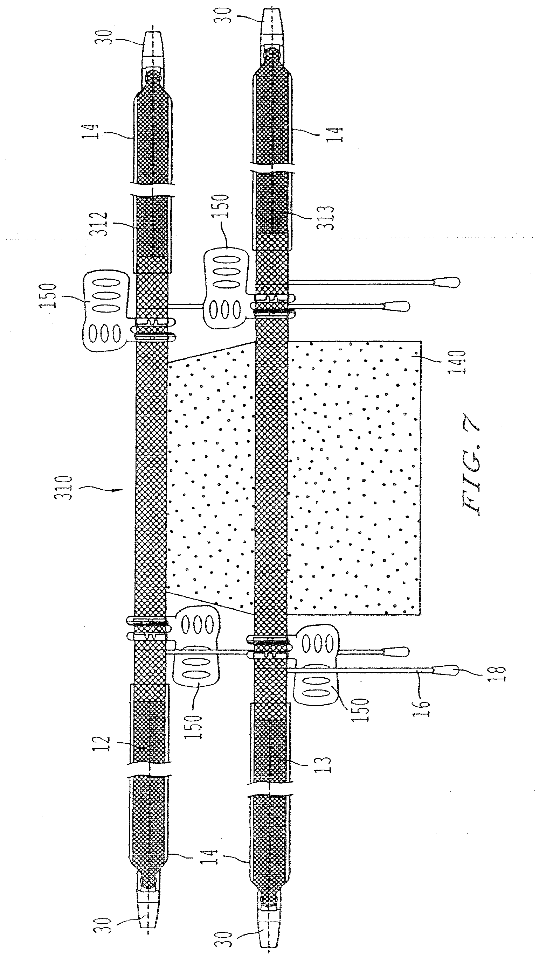

Figure 7 shows an alternate example of the surgical apparatus being part of the kit according to the present invention. Apparatus 310 includes straps 312 and 313 and biologic graft 140. Straps 12 and 13 have connectors 30 at each end thereof. Biologic graft 140 is connected to a central portion of each strap. In the embodiment shown in Figure 7, biologic graft 140 is connected to strap 12 and strap 13 at a portion equidistant from each end of the straps. It should be readily apparent to one skilled in the art that other configurations are possible, and these modifications are within the scope of the invention as claimed.

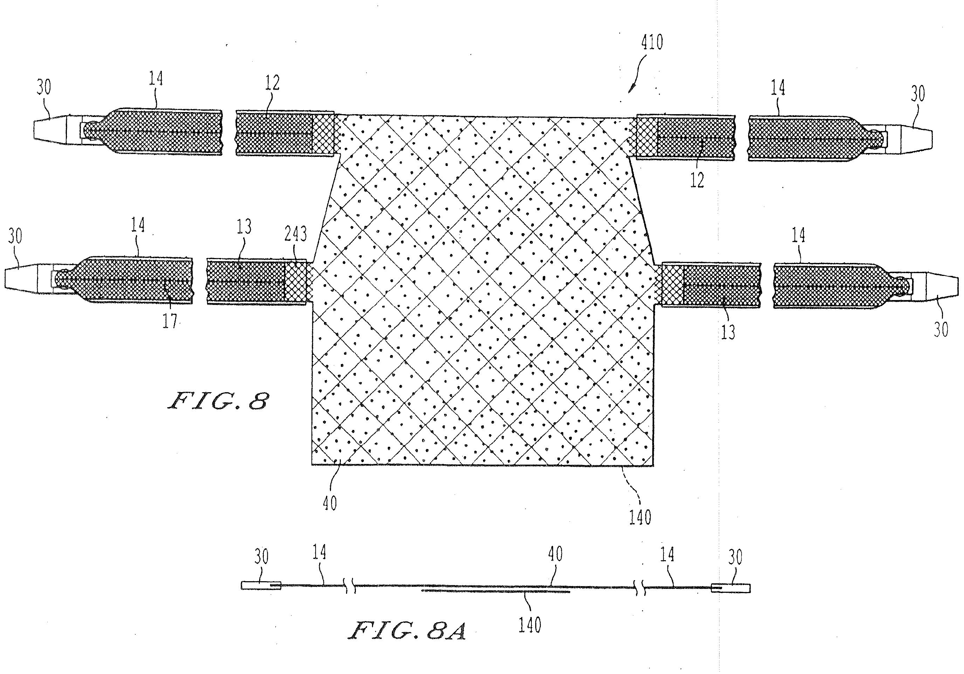

Figures 8 and 8A illustrate a surgical apparatus according to another alternate example being part of the kit according to the present invention. Apparatus 410 includes a biologic graft 140 and a synthetic support member 40. In the embodiment shown in Figures 8 and 8A, biologic graft 140 and synthetic support member 40 have the same area and are attached to overlie one another. However, it should be readily apparent to one skilled in the art that a surgical support apparatus having a biologic graft and a synthetic support member having different areas and/or offset from one another could be used, and these modifications are within the scope of the invention as claimed. Further, apparatus 410 may be implanted in the patient such that biologic graft 140 faces the bladder and support member 40 faces the vaginal wall, or such that biologic graft 140 faces the vaginal wall and support member 40 faces the bladder. The implantation configuration is based on the preference of the surgeon.

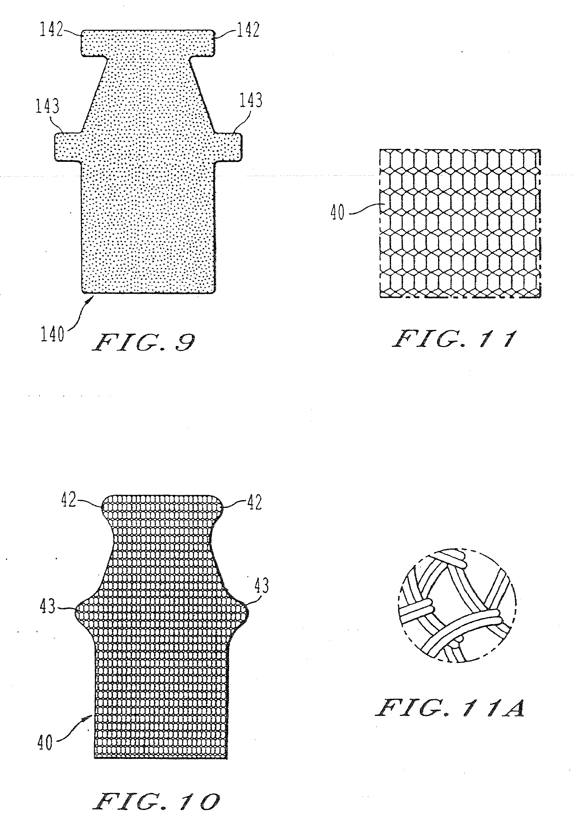

Figure 9 shows support member 140 made of a non-synthetic material. Suitable non-synthetic materials include allografts, homografts, heterografts, autologous tissues, cadaveric fascia, autodermal grafts, dermal collagen grafts, autofascial heterografts, whole skin grafts, porcine dermal collagen, lyophilized aortic homografts, preserved dural homografts, bovine pericardium and fascia lata. Member 140 includes tabs 142 and 143 for connecting to straps, as shown in Figure 5.

Figure 10 shows support member 40 made of a synthetic material. Member 40 includes tabs 42 and 43 for connecting to straps, as shown in Figure 1. Commercial examples of synthetic materials include Marlex™ (polypropylene) available from Bard of Covington, R. I., Prolene™ (polypropylene), Prolene Soft Polypropylene Mesh or Gynemesh (nonabsorbable synthetic surgical mesh), both available from Ethicon, of New Jersey, and Mersilene (polyethylene terphthalate) Hernia Mesh also available from Ethicon, Gore-Tex™ (expanded polytetrafluoroethylene) available from W. L. Gore and Associates, Phoenix, Ariz., and the polypropylene sling available in the SPARC™ sling system, available from American Medical Systems, Inc. of Minnetonka, Minn, Dexon™ (polyglycolic acid) available from Davis and Geck of Danbury, Conn., and Vicryl™ available from Ethicon.

Other examples of suitable materials include those disclosed in published U.S. patent application Ser. No. 2002/0072694. More specific examples of synthetic materials include, but are not limited to, polypropylene, cellulose, polyvinyl, silicone, polytetrafluoroethylene, polygalactin, Silastic, carbon-fiber, polyethylene, nylon, polyester (e.g. Dacron) polyanhydrides, polycaprolactone, polyglycolic acid, poly-L-lactic acid, poly-D-L-lactic acid and polyphosphate esters. See Cervigni et al., The Use of Synthetics in the Treatment of Pelvic Organ Prolapse, Current Opinion in Urology (2001), 11: 429-435.

Figures 11 and 11A illustrate two possible embodiments for the stitching of synthetic support member 40. However, it should be readily apparent to one skilled in the art that other knitting patterns are possible, and these modifications are within the scope of the invention as claimed.

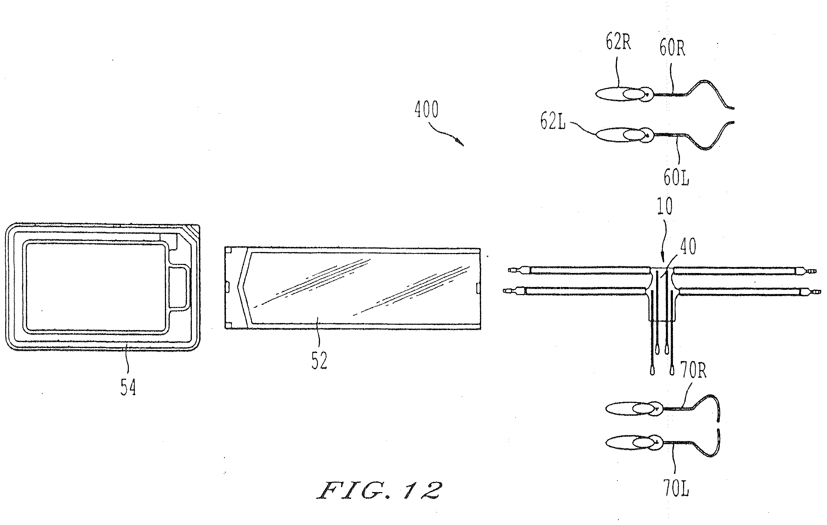

Referring to Figure 12, the present invention includes a surgical kit 400. The kit 400 preferably includes at least two superior needles 70R and 70L. Right superior needle 70R is configured to be held in the surgeon's right hand and such that the tip of the needle enters an incision on the left side of the patient where the left adductor longus tendon of the patient inserts into a left portion of the pubic ramus bone of the patient, lateral to the edge of the pubic ramus bone, and travels through the top of the left obturator foramen to exit through an incision in the vagina of the patient. Left superior needle 70L is configured to be held in the surgeon's left hand and such that the tip of the needle enters an incision on the right side of the patient where the right adductor longus tendon of the patient inserts into a right portion of the pubic ramus bone of the patient, lateral to the edge of the pubic ramus bone, and travels through the top of the right obturator foramen to exit through an incision in the vagina of the patient.

The needles included in the kit according to the kit according to the present invention may further include the needles described in published U.S. patent application Ser. Nos. 2002 -0065246-A1; 2002-0151762-A1; 2002-0147382-A1; 2002-0107430-A1, U.S. patent application Ser. No. 2002-0099258-A1 and U.S. patent application Ser. No. 2002-0099259-A1; and U.S. Provisional Application Serial Nos. 60/263,472, filed Jan. 23, 2001; 60/269,829, filed Feb. 20, 2001: 60/281,350, filed Apr. 4, 2001; 60/295,068, filed Jun. 1, 2001; 60/306,915, filed Jul. 20, 2001, and 60/332,330, filed Nov. 20, 2001. In an embodiment that is particularly suitable for a transobturator surgical procedure, the needles include needles as described in U.S. patent

The individual elements of the kits of the present invention may be packaged together as shown in Figure 12 with a cover 52 and tray 54. Alternatively, the individual elements may be separately packaged or packaged in subassemblies depending on a variety of factors such as shelf life and sterilization requirements. They may be assembled at the manufacturing location or at the healthcare location. Any suitable sterilization procedure may be utilized to sterilize the contents of a kit. Suitable sterilization techniques include, but are not limited to, steam, ethylene oxide, electron beam, vapor (e.g. hydrogen peroxide or peracetic acid), gamma or plasma procedures.

The kit shown in Figure 12 includes a support apparatus including a mesh support member 40. It should be readily apparent to one skilled in the art that kits using biological support members, as described above, may be made, and these modifications are within the scope of the invention as claimed. Further, a kit comprising a biologic graft may have the biologic graft pre-attached to the straps, or the graft may be separate from the straps and require the surgeon to attach the straps to the graft, as discussed below.

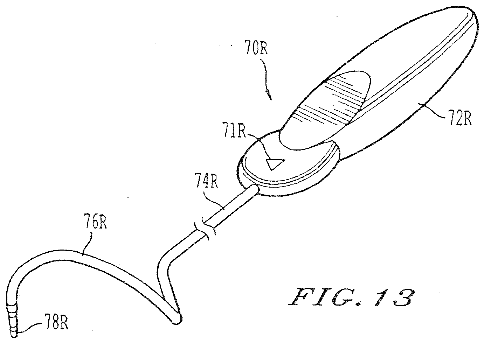

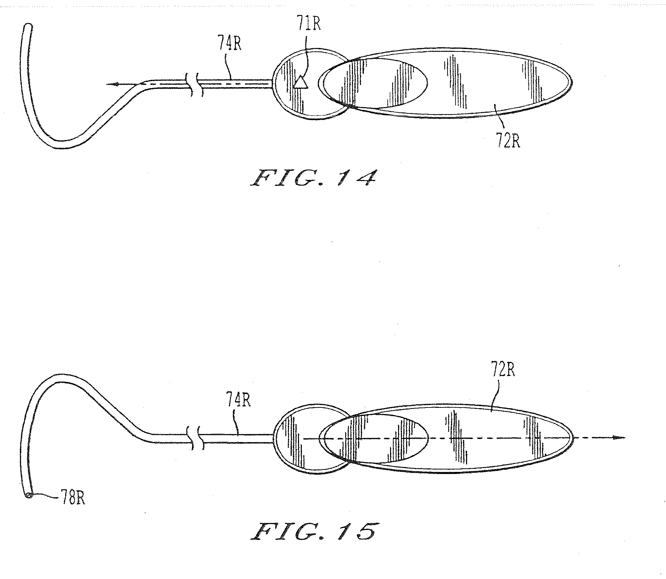

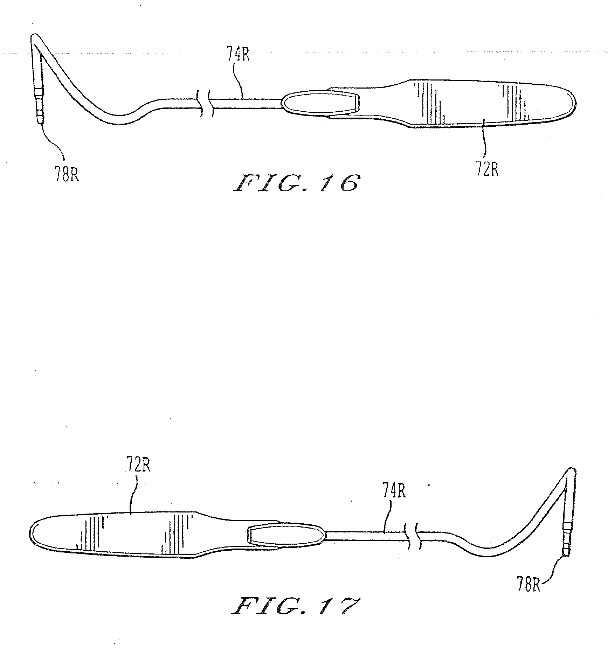

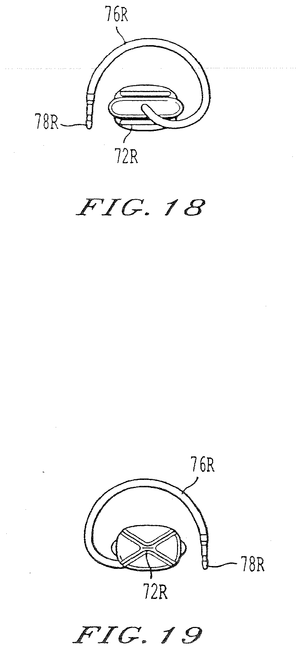

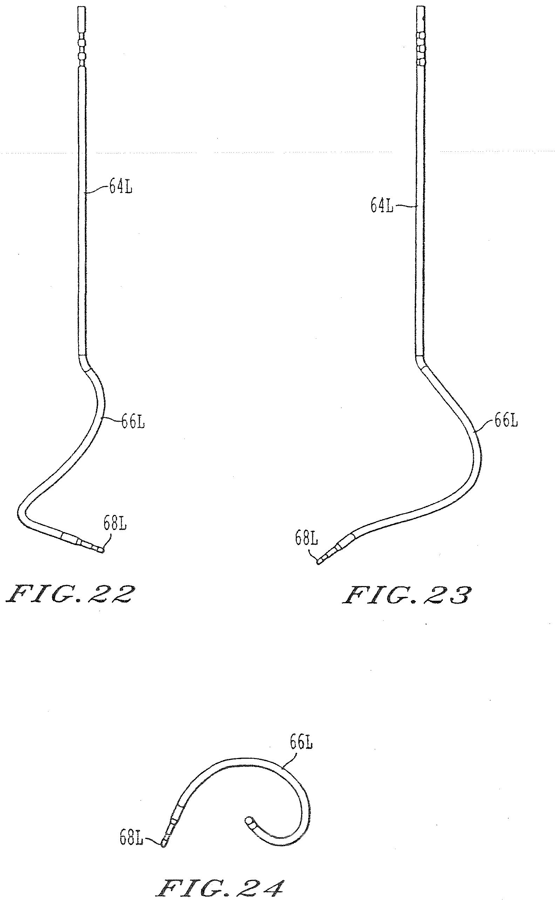

The kit shown in Figure 12 also includes four needles: right inferior needle 60R, left inferior needle 60L, right superior needle 70R, and left superior needle 70L. Embodiments of these needles are shown in Figures 13-24 and are described herebelow.

Figures 13-19 illustrate an embodiment of right superior needle 70R of the present invention. (Left superior needle 70L is a mirror image of the right superior needle 70R.) Right superior needle 70R includes indicia 71R, handle 72R, shaft 74R, curved portion 76R, and tip portion 78R. Indicia 71R designates whether the needle is the right or left needle by pointing to the surgeon's right or left side, as the surgeon holds the needle handle. (The surgeon's right side corresponds to the patient's left side.)

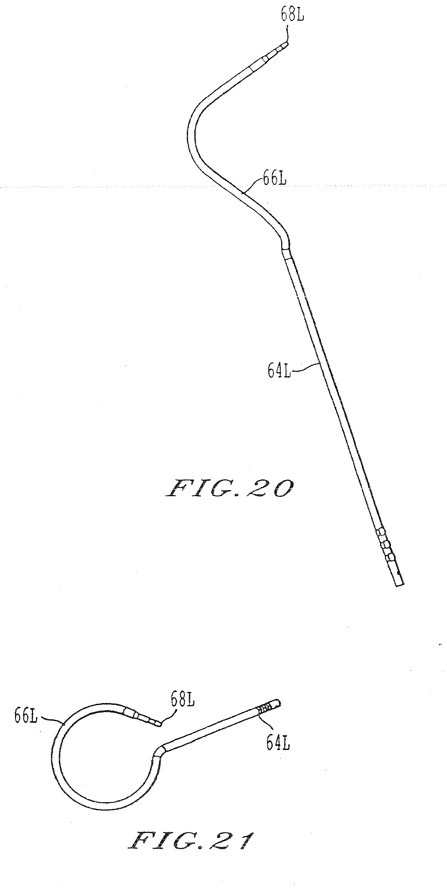

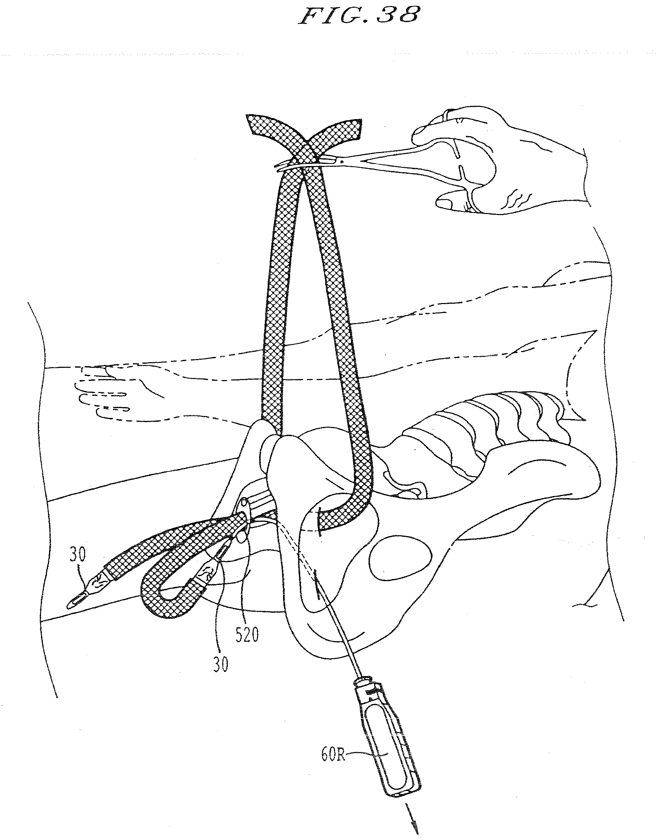

Figures 20-24 illustrate an exemplary shaft of left inferior needle 60L, without handle 62L. (Right inferior needle 60R is a mirror image of the left inferior needle 60L.) Left inferior needle 60L includes a handle 62L, a shaft 64L, a curved portion 66L, and a tip portion 68L. Left inferior needle 60L is configured to be held in a surgeon's left hand such that tip 68L enters an incision 530L on the right side of the patient where a right inferior edge of the pubic ramus bone of the patient ends at a bottom of the right obturator foramen of the patient, and travels through the right obturator foramen to exit through an incision in the vagina of the patient. Right inferior needle 60R is configured to be held in a surgeon's right hand such that tip 68R enters an incision on the left side of the patient where a left inferior edge of the pubic ramus bone of the patient ends at a bottom of the left obturator foramen of the patient, and travels through the left obturator foramen to exit through an incision in the vagina of the patient. This is shown in Figure 38.

The above-described needles may be disposable or reusable.

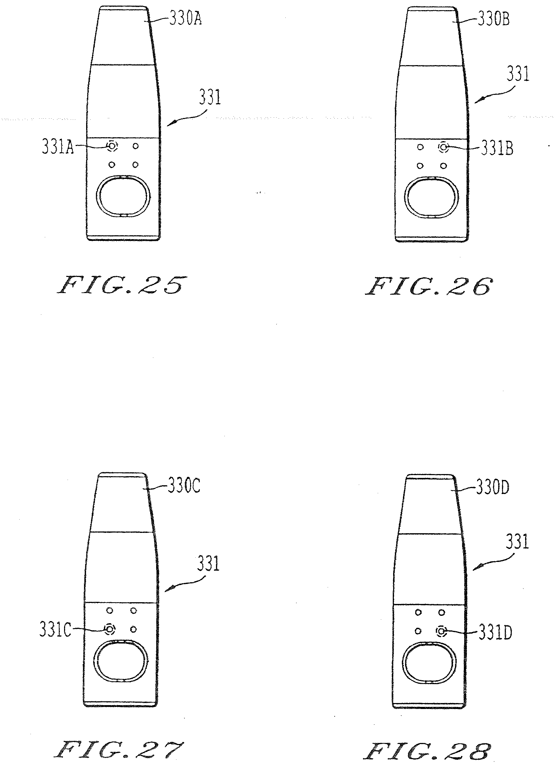

Figures 25-28 show connectors to be part of the kit of the present invention having indicia thereon. Figure 25 shows left superior connector 330A having indicia 331. Indicia 331 includes symbol 331A indicating that the connector is the left superior connector. Figure 26 shows right superior connector 330B having indicia 331. Indicia 331 includes symbol 331B indicating that the connector is the right superior connector. Figure 27 shows left inferior connector 330C having indicia 331. Indicia 331 includes symbol 331C indicating that the connector is the left inferior connector. Figure 28 shows right inferior connector 330D having indicia 331. Indicia 331 includes symbol 331D indicating that the connector is the right inferior connector. Right connectors are located on the surgeon's right side and left connectors are located on the surgeon's left side.

Figures 25-28 show connectors including symbolic indicia to identify each connector. It should be readily apparent to one skilled in the art that other symbols, markings, or words could be used to identify the connectors, and that these modifications are within the scope of the invention as claimed.

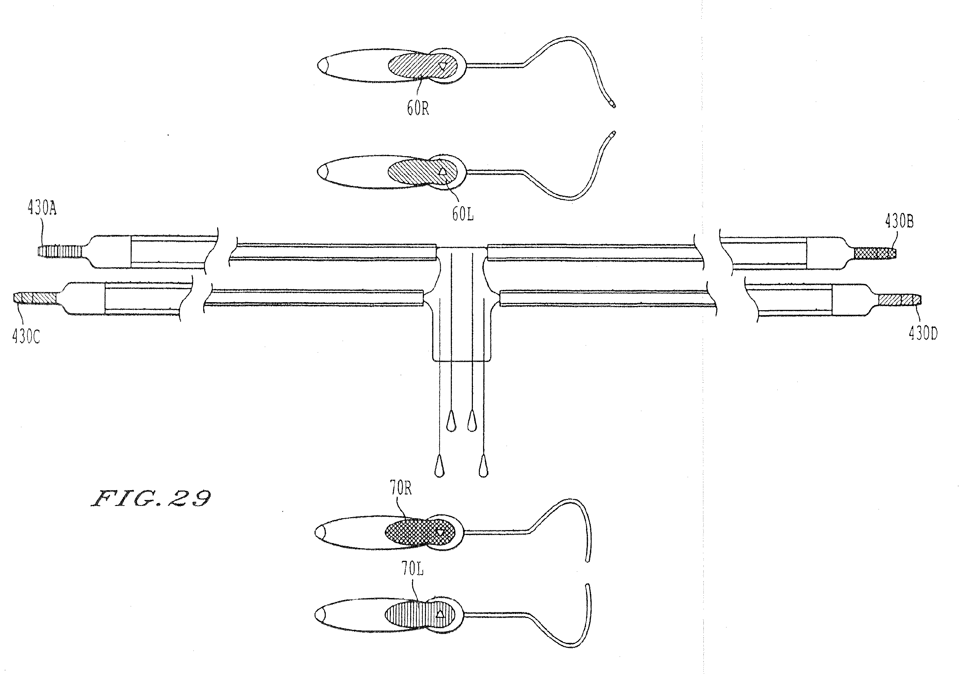

Figure 29 shows another embodiment of the present invention wherein connectors and the handles of the corresponding needles are matching colors. For example, the color of the handle of needle 70L matches the color of connector 430A. The color of the handle of needle 70R matches the color of connector 430B. The color of the handle of needle 60R matches the color of connector 430D. The color of the handle of needle 60L matches the color of connector 430C.

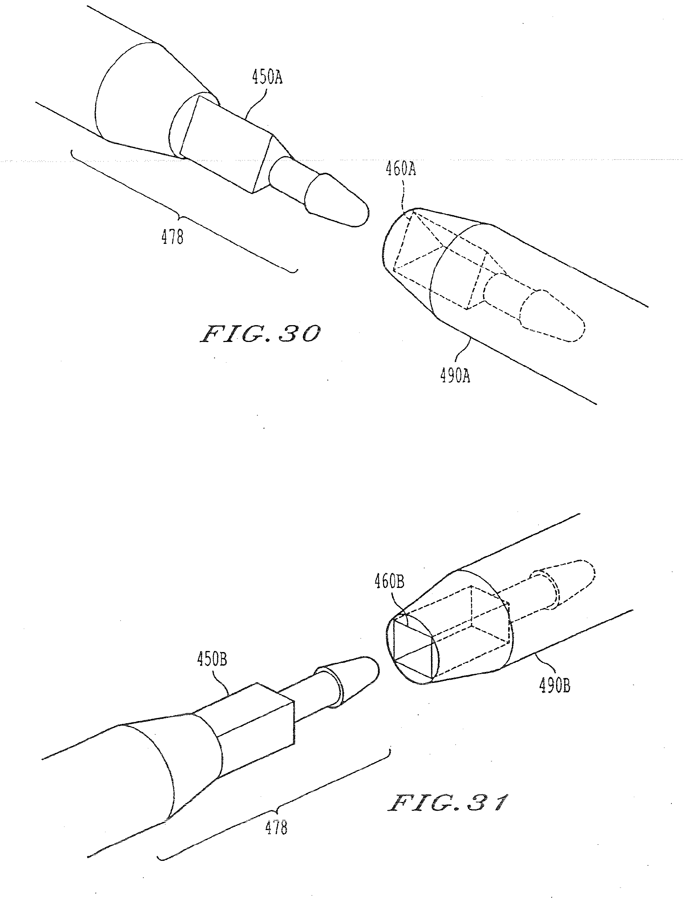

Figures 30 and 31 are perspective views of a needle tips having a cross sections that are configured to match the cross sections of a connector aperture. Figure 30 shows that the cross section of portion 450A of needle tip portion 478A is a triangle. The cross section of portion 450A matches triangle shaped aperture 460A in connector 490A. Figure 31 shows that the cross section of portion 450B of needle tip portion 478B is a square. The cross section of portion 450B matches square shaped aperture 460B in connector 490B.

In one embodiment, each needle tip has a cross section that matches the cross section of an aperture of the corresponding connector, and the tip cross section is incompatible with the other connector apertures. For example, the cross section of the portion 450A, a triangle, would not fit in aperture 460B, a square, and vice versa. Thus, even if the connectors are confused, it is physically impossible for a surgeon to insert the needle tip in the incorrect connector without damaging the tip or connector. It should be readily apparent to one skilled in the art that other shaped tips and apertures are possible, and these modifications are within the scope of the invention as claimed.

### Example of a Surgical Procedure

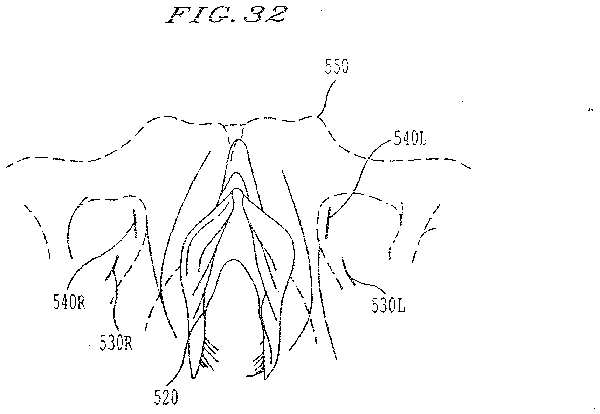

The following description, illustrated in Figures 32-39, is an exemplary method for using the disclosed surgical support apparatus 10 having a mesh support member 40. It should be readily apparent to those skilled in the art that modifications may be made to the following method.

If the embodiment of the surgical support apparatus 110 including a biological support member 140 is used, the biological support member 140 must be prepared before making the vaginal incision. Instructions for preparing the biological support member are given after the present description.

In preparation for surgery, the patient is placed in a modified dorsal lithotomic position with hips flexed, legs elevated in stirrups and buttocks even with edge of the surgical table. The patient's bladder is emptied. A catheter is not required during the procedure, but may aid in identifying the urethra during the procedure. A weighted vaginal retractor or other suitable vaginal retraction is used, if desired.

The length of the vaginal incision is marked with a skin pencil starting below the bladder neck, over the most prominent part of the prolapse, to the lowermost part of the prolapse. (Variations may occur in specific incisions due to individual technique and patient anatomy.) An incision is made over this marking. The incision site may be infiltrated with saline, if desired. An Allis forceps is placed on the incision margin to expose the incision. The patient's bladder is dissected off the vagina up to the lateral sulcus and posterior to the vaginal vault. This dissection allows palpation of the medial edge of the inferior pubic ramus, assisting in guiding the superior and inferior needles to the exit points free from the bladder. The patient's cystocele is then reduced using midline plication, if desired.

Next, markings are made to identify the locations for needle entry incisions. The vaginal dissection is completed prior to marking needle entry incisions to allow for digital palpation along the ischiopubic ramus. The needle entry points are palpated internally and externally with the thumb and index finger before marking, as discussed hereafter.

The edge of the ischiopubic ramus beginning at the level of the vaginal incision is palpated, continuing along the edge of the bone cephalad toward the level of the clitoris denoting where the adductor longus tendon inserts into the pubic ramus. The superior skin incisions are marked approximately at this location and lateral to the edge of the bone. The markings are made according to the same method on both sides (right and left) of the patient's body. Both marks lie in a straight line at the approximate level of the clitoris. The edge of the inferior pubic ramus is palpated until it ends at the bottom of the obturator foramen. The inferior skin incisions are then marked. The inferior skin incisions are located at a point approximately 3 centimeters below and 2 centimeters lateral to the superior marks. Again, the markings are made according to the same method on both sides of the patient's body.

A small vertical stab incision is made over all four markings to provide needle entry incisions. Right superior incision 540R, left superior incision 540L, right inferior incision 530R, and left inferior incision 530L are all shown in Figure 32. (Right and left with regard to the incisions are the patient's right and left sides.)

The above-described surgical kit is opened. The package integrity is checked to ensure that the kit was not compromised in shipping, and the components of the kit are inspected for damage.

The following method describes the straps on the surgeon's right side (the patient's left side) being surgically installed before the straps on the surgeon's left side (the patient's right side). However, it should be readily apparent to one skilled in the art that the straps of either side could be installed first.

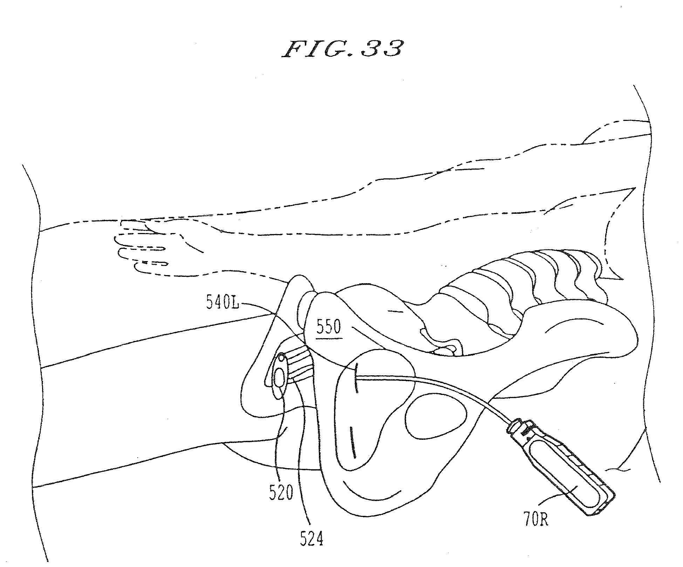

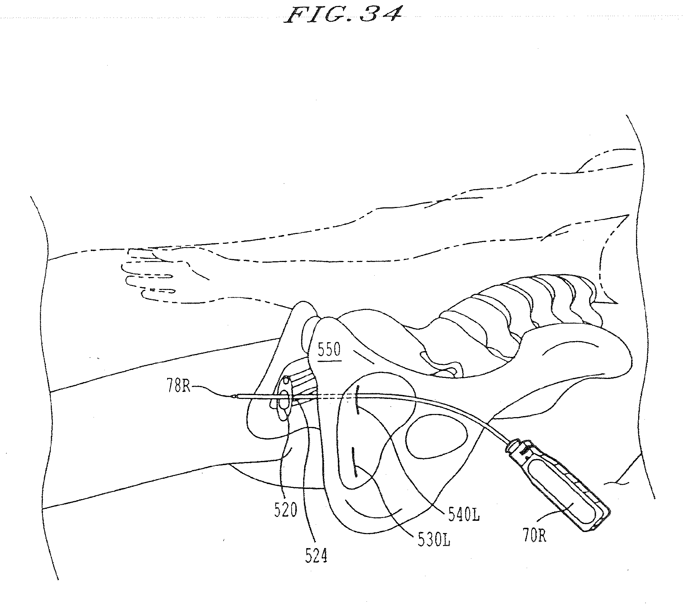

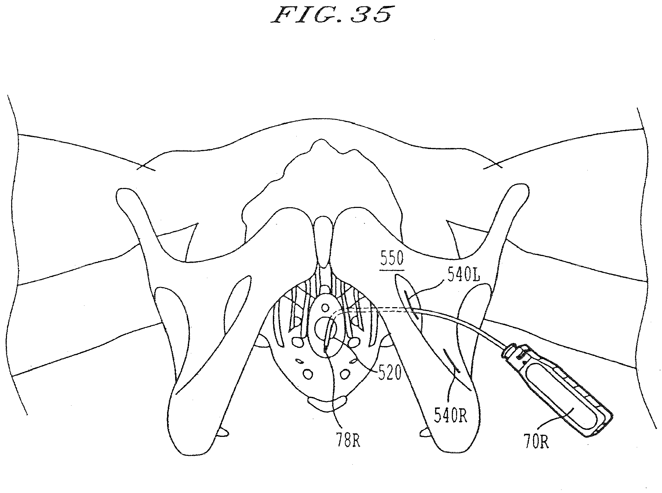

Tip 78R of right superior needle 70R is now inserted through left superior incision 540L, through the left obturator foramen, and then through the vaginal incision 524. Tip of right superior needle 70R is pointed perpendicular to the skin with tip 78R in the left superior incision 540L, shown in Figure 33. The thumb from the surgeon's right hand is on the outside curve of needle to control the needle movement as it perforates the obturator membrane and muscle. The right thumb pushes the needle through the obturator muscle and membrane. The needle shaft and handle is positioned at a 45° angle to the patient's vertical axis and close to the patient's body. The needle handle is rotated to move the needle tip and curve around the posterior surface of the ischial pubic ramus toward the vaginal incision and index finger. (If the needle tip hits the pubic bone during rotation, the needle is retracted. The needle tip is then penetrated beyond initial insertion depth and rotate again toward the vaginal incision.) The needle tip is palpated with the surgeon's finger. The finger meets the needle tip as it moves around the pubic ramus. (If the needle tip can not be located, the needle tip is retracted to just behind the pubic ramus and advanced again.) The needle tip is guided by the surgeon with the surgeon's finger towards the vaginal incision until the needle tip extends through the vaginal incision, shown in Figures 34 and 35.

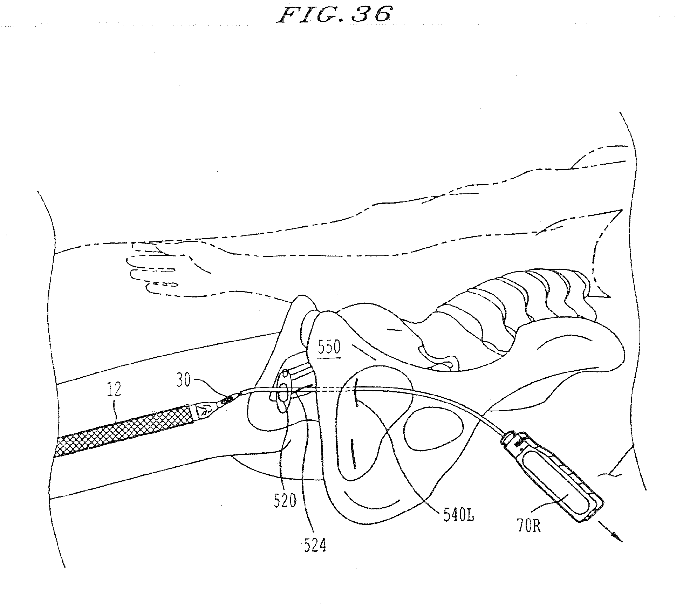

The support member is then oriented so that the tail of the graft is pointing away from the surgeon. (The marking indicia disclosed herein may be used to determine the correct orientation of the support member.) The right superior connector is connected to the tip of the right superior needle, the tip extending out of the vaginal incision, as shown in Figure 36. The superior needle connectors are closest to the leading edge of the graft that will be below the bladder neck.

Before attaching the connectors, the surgeon ensures that the self-fixating mesh and graft are not twisted, as the connectors are not removable once snapped onto the needle. Once the connector is attached to the needle, the needle is rotated back through the skin incision pulling the connector and associated plastic insertion sheath and graft into position.

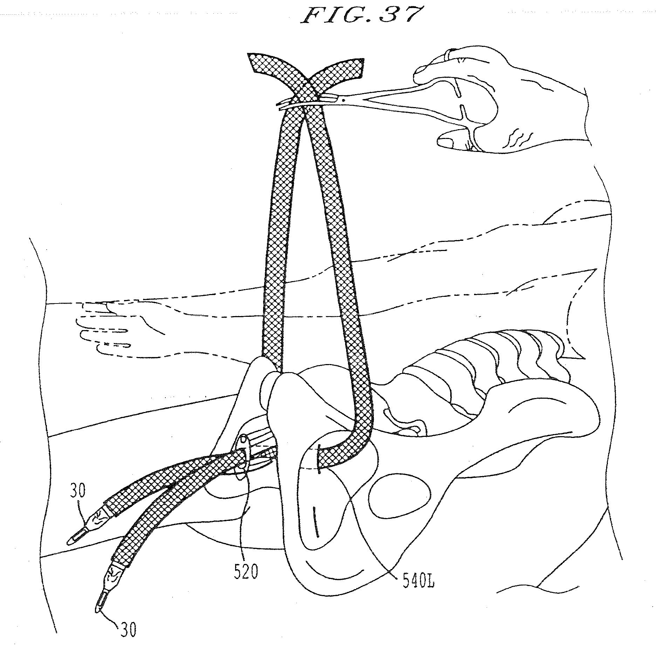

The above process is repeated with the left needle on the patient's right side. The partially implanted apparatus is shown in Figure 37, with superior straps and support member 40 implanted and the inferior straps extending outside the body through the vaginal incision.

The insertion sheaths and mesh are then cut below the blue mark on the end portion of the plastic sheath and discarded. This step allows the sheath to slide freely relative to the mesh. The sheaths are not removed at this time.

The tip of the right inferior needle is now inserted through left inferior incision 530L, through the left obturator foramen, and then through the vaginal incision. The tip of the right inferior needle is pointed perpendicular to the skin with the tip in the left inferior incision. The exit point for the needle is confirmed to be clear of the bladder wall by the surgeon placing their right index finger at the distal end of the vaginal incision and visualizing where needle exits the distal end of vaginal incision. The surgeon's right thumb is on the outside curve of needle to control the needle movement as it perforates the obturator membrane and muscle. The right thumb pushes the needle through the obturator muscle and membrane.

The needle shaft and handle is positioned parallel to the patient's vertical axis and close to the patient's body. The needle handle is rotated, moving the needle tip and curve toward the distal end of the vaginal incision. The surgeon is careful to avoid buttonholing the fornix to prevent bleeding. The needle tip is palpated as it moves through the distal end of the vaginal incision. The right inferior needle tip is shown extending outside the vaginal incision in Figure 38.

The right inferior connector is connected to the right inferior needle tip. Again, before attaching the connectors, the surgeon ensures that the self-fixating mesh and graft are not twisted, as the connectors are not removable once snapped onto the needle. The needle is rotated back through the skin incision pulling the connector and associated plastic insertion sheath and graft into position.

The above process is repeated with the left inferior needle on the patient's right side.

The insertion sheath and mesh are then cut below the blue mark on the end portion of the plastic sheath and discarded. This step allows the sheath to slide freely relative to the mesh. The sheaths are not removed at this time.

A cystoscopy is done to check the integrity of the ureters and bladder.

Any vaginal retraction is now removed to allow adjusting the tension of the mesh to reduce bladder bulge. The surgeon confirms the mesh is lying flat and not overlapping under the vaginal wall. The superior leading edge of the support member should be positioned below the bladder neck without tension. The inferior tail portion of the support member should be positioned at the distal end of the vaginal incision or towards the vaginal apex without tension.

If the mesh needs to be loosened, an instrument is placed between the mesh and vaginal wall and pulled down, or away from the vaginal wall until proper tension is achieved.

Each of the four plastic sheaths are removed and discarded, while ensuring the support member graft is not being over tensioned. Once the plastic sheaths are removed, further adjustment is minimized.

If the mesh needs to be tightened, the tensioning suture exiting the skin incision on each side is grasped using a hemostat. The suture is wrapped around the hemostat to improve the grip and pulled up or out to tighten until proper tension is achieved.

To loosen a biologic graft, the surgeon uses a hemostat or a clamp to pull from each of the hanging loosening sutures. The surgeon uses the clamps to pull down and loosen the strap mesh as desired. The surgeon is careful not to pull on tab 18 on loosening suture 16 to loosen the strap mesh.

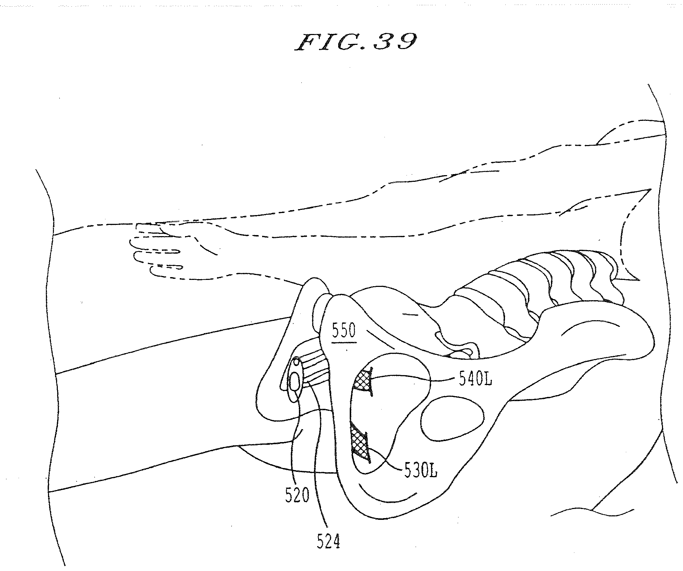

The surgeon cuts one end of each loosening sutures and pulls tab 18 until the entire loosening suture is removed. The mesh is then trimmed at the level of the subcutaneous tissue and all five incisions are closed. Excess vaginal tissue may be excised. Variations of this step may occur due to individual technique and patient anatomy. The final implanted apparatus is shown in Figure 39.

After the operation, a catheter and/or vaginal pack can be used at the discretion of the surgeon. It is removed prior to discharge. Antibiotic prophylaxis should be given. The ability of the patient to empty the bladder should be confirmed prior to discharge.

If a biologic graft is used, the following steps are performed before making the vaginal incision. The biologic graft is removed from the package and prepared per included instructions, if needed. A precut biologic is prepared by orienting the graft with the tail portion pointing at the surgeon. Beginning with the right or left inferior landing tabs on the biologic graft (closest to the surgeon), the graft is attached to clamps 150. Attaching the inferior tabs first allows space to attach the two superior mesh appendages with the clamp. The clamps are squeezed to separate mesh tape. The graft material is inserted into the open clamp using printed marks as guides to center the graft. (The printed side of the plastic sheath is facing the surgeon as the surgical apparatus is placed in the body.) The clamp is released to secure graft material. A desired suture is passed up through the clamp using a suturing mark as a guide. The suture is then passed down using the opposite suturing mark as the guide. The passed sutures are then secured using the surgeon's knot(s) of choice. Additional throws are made if needed. The clamp sutures are cut by passing a scissors or a scalpel down the scissors slot on each side of the clamp. The clamps are then removed. The clamp attachment sutures remain with the clamp. The surgeon assesses the attachment of the graft material mesh tape. The protective sheath is slid over the mesh connection to aid deployment.

The preceding steps are repeated on the opposite side of the graft. The sutures are passed such that the attachment knots are all on the same side of the graft. The biologic is placed in a saline bath to keep it hydrated during the remainder of the procedure. The graft tail is trimmed at the time of vaginal marking and dissection to reflect patients anatomy, if needed.

In addition, when using the biologic graft, the surgeon is careful when drawing the strap through the body that the sheath covers the graft connections and that the graft material and graft connections are not damaged.

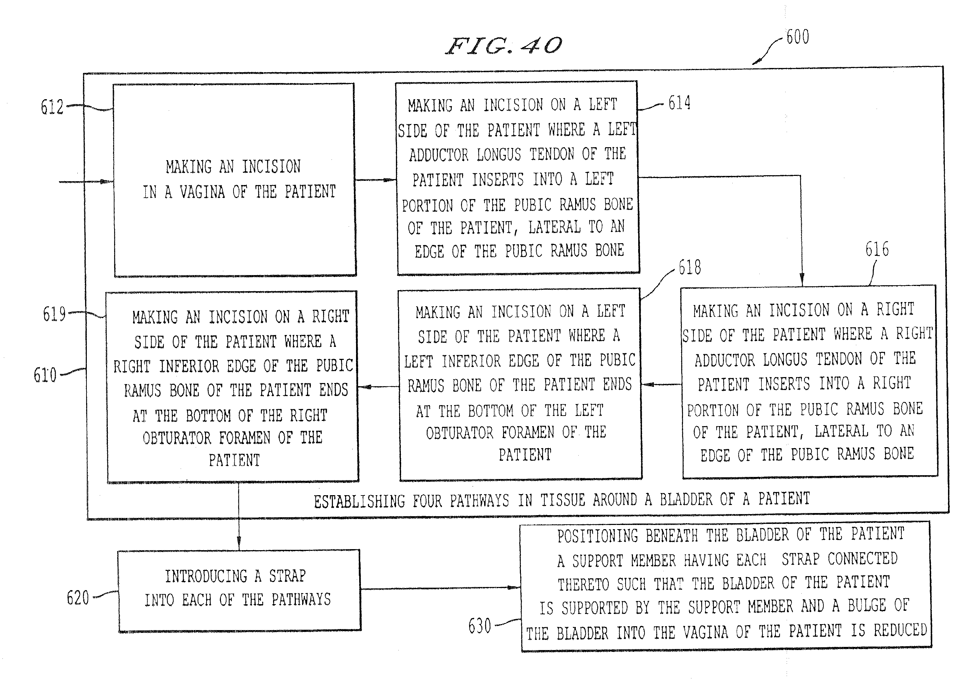

Figure 40 illustrates a method of practicing the present disclosure Method 600 includes the steps of: establishing four pathways in tissue around a bladder of a patient (step 610), introducing a strap into each of the pathways (step 620), and positioning beneath the bladder of the patient a support member having each strap connected thereto such that the bladder of the patient is supported by the support member and a bulge of the bladder into the vagina of the patient is reduced (step 630). Step 610 comprises the steps of: making an incision in the vagina of the patient (step 612), making an incision on a left side of the patient where a left adductor longus tendon of the patient inserts into a left portion of the pubic ramus bone of the patient, lateral to an edge of the pubic ramus bone (step 614), making an incision on a right side of the patient where a right adductor longus tendon of the patient inserts into a right portion of the pubic ramus bone of the patient, lateral to an edge of the pubic ramus bone (step 616), making an incision on a left side of the patient where a left inferior edge of the pubic ramus bone of the patient ends at the bottom of the left obturator foramen of the patient (step 618), and making an incision on a right side of the patient where a right inferior edge of the pubic ramus bone of the patient ends at the bottom of the right obturator foramen of the patient (step 619).

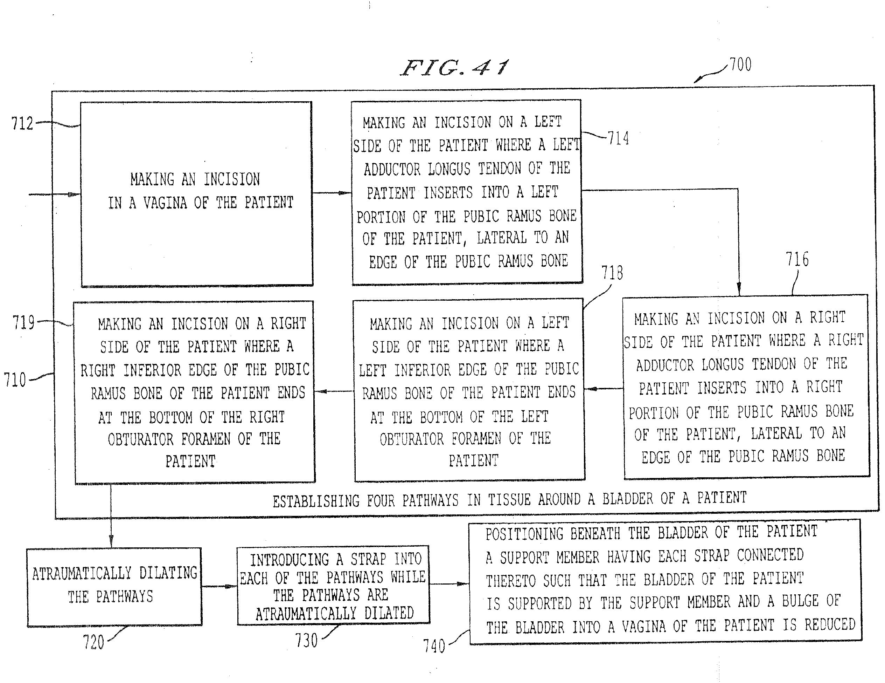

Figure 41 illustrates an alternate method of practicing the present disclosure. Method 700 includes the steps of: establishing four pathways in tissue around a bladder of a patient (step 710), atraumatically dilating the pathways (step 720), introducing a strap into each of the pathways while the pathways are atraumatically dilated (step 730), and positioning beneath the bladder of the patient a support member having each strap connected thereto such that the bladder of the patient is supported by the support member and a bulge of the bladder into the vagina of the patient is reduced (step 740). Step 710 comprises the steps of: making an incision in the vagina of the patient (step 712), making an incision on a left side of the patient where a left adductor longus tendon of the patient inserts into a left portion of the pubic ramus bone of the patient, lateral to an edge of the pubic ramus bone (step 714), making an incision on a right side of the patient where a right adductor longus tendon of the patient inserts into a right portion of the pubic ramus bone of the patient, lateral to the edge of the pubic ramus bone (step 716), making an incision on a left side of the patient where a left inferior edge of the pubic ramus bone of the patient ends at the bottom of the left obturator foramen of the patient (step 718), and making an incision on a right side of the patient where a right inferior edge of the pubic ramus bone of the patient ends at the bottom of the right obturator foramen of the patient (step 719).

## Claims

1. A kit (400) for repairing cystocele comprising a support apparatus (410) and at least two needles (70R,70L),
wherein:
the support apparatus comprises:
a substantially rectangular support member (40) having two short sides and two long sides, a first end of said rectangular member at a first of said two short sides, a second end of said rectangular member at a second of said two short sides;
a first strap (12) connected to a first long side proximate said first end of said member;
a second strap (12) connected to a second long side proximate said first end of said member;
a third strap (13); and
a fourth strap (13); each of said straps comprising a connector (30) configured to be coupled with a tip of a needle (78) **characterized in that**
the first strap is a first inferior strap;
the second strap is a second inferior strap,
the third strap is a third inferior strap connected to a first long side proximate a middle portion of said first long side; and
the fourth strap is a fourth inferior strap connected to a second long side proximate a middle portion of said second long side,
a first three-dimensionally curved needle (60R) configured to extend from an incision on a right side of a patient where a right adductor longus tendon of said patient inserts into a right portion of a pubic ramus bone of said patient, lateral to an edge of said pubic ramus bone, through a right obturator foramen of said patient, to an incision in a vagina of said patient;
a second three-dimensionally curved needle (60L) configured to extend from an incision on a left side of said patient where a left adductor longus tendon of said patient inserts into a left portion of said pubic ramus bone of said patient, lateral to an edge of said pubic ramus bone, through a left obturator foramen of said patient, to said incision in said vagina of said patient;
a third three-dimensionally curved needle (60R) configured to extend from an incision on said right side of said patient where a right inferior edge of said pubic ramus bone of said patient ends at a bottom of said right obturator foramen of said patient, through said right obturator foramen of said patient, to said incision in said vagina of said patient; and
a fourth three-dimensionally curved needle (60L) configured to extend from an incision on said left side of said patient where a left inferior edge of said pubic ramus bone of said patient ends at a bottom of said left obturator foramen of said patient, through said left obturator foramen of said patient, to said incision in said vagina of said patient.

2. The kit recited in claim 1 wherein said support apparatus comprises a biologic graft.

3. The kit recited in claim 2 wherein said biologic graft is pre-attached to each of said straps.

4. The kit recited in claim 1 wherein said support member is knitted, and said support apparatus comprises a biologic graft fixed over the knitted support member.

5. The kit recited in claim 1 wherein said support member is knitted with a first bar setting; and wherein said straps are continuously knitted with said support member, and said straps are knitted with a second bar setting.

6. The kit recited in claim 5 wherein
said first strap is continuously knitted with said first long side proximate said first end of said member;
said second strap continuously knitted with said second long side proximate said first end of said member;
said third strap continuously knitted with said first long side proximate a middle portion of said first long side; and
said fourth strap continuously knitted with said second long side proximate a middle portion of said second long side.

7. The kit recited in claim 5 wherein said first bar setting is:
Bar 1: 1/0, 2/3, 2/1, 2/3, 1/0, 1/2, 1/0, 1/2;
Bar 2: 1/0, 2/3, 2/3, 1/0; and
Bar 3: 2/3, 1/0, 1/2, 1/0, 2/3, 2/1, 2/3, 2/1.

8. The kit recited in claim 1 or 7 wherein each connector has an aperture configured to receive a tip of a needle, each connector aperture having a different shape; and wherein
each needle has a tip having a shape configured to mate with only one connector aperture.

9. The kit recited in claim 1 wherein each strap or connector has identifying indicia thereon.

10. The kit recited in claim 1 wherein each connector has a color; and wherein each needle has a color matching a color of a corresponding connector.

11. A kit according to any of claims 1 through 10 wherein each strap comprises a tensioning suture.

12. A kit according to any of claims 1 through 11 wherein each strap is covered by a sheath.

13. A kit according to any of claims 1 through 12 wherein each needle is attached to a handle and each handle comprises a length, a longitudinal axis along the length, opposing major surfaces along the length, opposing minor sides, and a cross section of each handle along the longitudinal axis exhibits an elongate, non-circular form.

## Patentansprüche

1. Set (400) zum Reparieren von Zystozelen mit einer Stützvorrichtung (410) und mindestens zwei Nadeln (70R, 70L), wobei:
die Stützvorrichtung aufweist:
ein im Wesentlichen rechteckiges Stützelement (40) mit zwei kurzen und zwei langen Seiten, wobei sich ein erstes Ende des rechteckigen Elements an einer ersten der zwei kurzen Seiten befindet und ein zweites Ende des rechteckigen Elements an einer zweiten der zwei kurzen Seiten befindet;
ein erstes Band (12), das mit einer ersten langen Seite nahe dem ersten Ende des Elements verbunden ist;
ein zweites Band (12), das mit einer zweiten langen Seite nahe dem ersten Ende des Elements verbunden ist;
ein drittes Band (13); und
ein viertes Band (13);
wobei jedes der Bänder einen Verbinder (30) aufweist, der ausgebildet ist, mit einer Spitze einer Nadel (78) gekoppelt zu werden;
**dadurch gekennzeichnet, dass**
das erste Band ein erstes unteres Band ist,
das zweite Band ein zweites unteres Band ist,
das dritte Band ein drittes unteres Band ist, das mit einer ersten langen Seite nahe eines mittleren Abschnitts der ersten langen Seite verbunden ist, und
das vierte Band ein viertes unteres Band ist, das mit einer zweiten langen Seite nahe eines mittleren Abschnitts der zweiten langen Seite verbunden ist,
eine erste dreidimensional gekrümmte Nadel (60R), die ausgebildet ist, sich von einer Inzision an einer rechten Seite eines Patienten, wo eine rechte Adductorlongus-Sehne des Patienten in einen rechten Abschnitt eines Schambeinknochens des Patienten eintritt, seitlich von einer Kante des Schambeinknochens durch ein rechtes Foramen obturatum des Patienten zu einer Inzision in einer Vagina des Patienten zu erstrecken;
eine zweite dreidimensional gekrümmte Nadel (60L), die ausgebildet ist, sich von einer Inzision an einer linken Seite des Patienten, wo eine linke Adductor-longus-Sehne des Patienten in einen linken Abschnitt des Schambeinknochens des Patienten eintritt, seitlich von einer Kante des Schambeinknochens durch ein linkes Foramen obturatum des Patienten zu der Inzision in der Vagina des Patienten zu erstrecken;
eine dritte dreidimensional gekrümmte Nadel (60R), die ausgebildet ist, sich von einer Inzision an der rechten Seite des Patienten, wo eine rechte untere Kante des Schambeinknochens des Patienten an einem untersten Teil des rechten Foramen obturatum des Patienten endet, durch das rechte Foramen obturatum des Patienten zu der Inzision in der Vagina des Patienten zu erstrecken; und
eine vierte dreidimensional gekrümmte Nadel (60L), die ausgebildet ist, sich von einer Inzision an der linken Seite des Patienten, wo eine linke untere Kante des Schambeinknochens des Patienten an einer untersten Seite des linken Foramen obturatum des Patienten endet, durch das linke Foramen obturatum des Patienten zu der Inzision in der Vagina des Patienten zu erstrecken.

2. Set nach Anspruch 1, wobei die Stützvorrichtung ein biologisches Implantat aufweist.

3. Set nach Anspruch 2, wobei das biologische Implantat vorher auf jedes der Bänder aufgebracht ist.

4. Set nach Anspruch 1, wobei das Stützelement geknüpft ist und die Stützvorrichtung ein biologisches Implantat aufweist, das über dem geknüpften Stützelement befestigt ist.

5. Set nach Anspruch 1, wobei das Stützelement in einer ersten Netzstruktur geknüpft ist, und wobei die Bänder kontinuierlich mit dem Stützelement verknüpft sind und die Bänder in einer zweiten Netzstruktur geknüpft sind.

6. Set nach Anspruch 5, wobei
das erste Band kontinuierlich mit der ersten langen Seite nahe dem ersten Ende des Elements verknüpft ist,
das zweite Band kontinuierlich mit der zweiten langen Seite nahe dem ersten Ende des Elements verknüpft ist,
das dritte Band kontinuierlich mit der ersten langen Seite nahe einem mittleren Abschnitt der ersten langen Seite verknüpft ist, und
das vierte Band kontinuierlich mit der zweiten langen Seite nahe einem mittleren Abschnitt der zweiten langen Seite verknüpft ist.

7. Set nach Anspruch 5, wobei die erste Netzstruktur folgendermaßen ist:
Netz 1: 1/0, 2/3, 2/1, 2/3, 1/0, 1/2, 1/0, 1/2,
Netz 2: 1/0, 2/3, 2/3, 1/0, und
Netz 3: 2/3, 1/0, 1/2, 1/0, 2/3, 2/1, 2/3, 2/1.

8. Set nach Anspruch 1 oder 7, wobei jeder Verbinder eine Öffnung aufweist, die ausgebildet ist, eine Spitze einer Nadel aufzunehmen, wobei jede Verbinderöffnung eine unterschiedliche Form hat, und wobei
jede Nadel eine Spitze hat, die eine Form hat, die ausgebildet ist, zu nur einer Verbinderöffnung zu passen.

9. Set nach Anspruch 1, wobei jedes Band oder jeder Verbinder Identifizierungsmerkmale aufweist.

10. Set nach Anspruch 1, wobei jeder Verbinder eine Farbe hat und wobei jede Nadel eine Farbe hat, die zur Farbe eines entsprechenden Verbinders passt.

11. Set nach einem der Ansprüche 1 bis 10, wobei jedes Band eine Spannnaht aufweist.

12. Set nach einem der Ansprüche 1 bis 11, wobei jedes Band von einer Ummantelung umgeben ist.

13. Set nach einem der Ansprüche 1 bis 12, wobei jede Nadel an einem Handstück befestigt ist und jedes Handstück eine Länge, eine Längsachse entlang der Länge, gegenüberliegende Hauptflächen entlang der Länge und gegenüberliegende Nebenseiten aufweist, und ein Querschnitt jedes Handstücks entlang der Längsachse eine längliche, nicht kreisförmige Form hat.

## Revendications

1. Ensemble (400) pour la réparation d'une cystocèle, comprenant un dispositif de support (410) et au moins deux aiguilles (70R,70L), où :
le dispositif de support comprenant :
un élément de support (40) sensiblement rectangulaire avec deux largeurs et deux longueurs, une première extrémité de l'élément rectangulaire sur une première des deux largeurs, une deuxième extrémité de l'élément rectangulaire sur une deuxième des deux largeurs ;
une première bande (12) raccordée à une première longueur à proximité de la première extrémité de l'élément ;
une deuxième bande (12) raccordée à une deuxième longueur à proximité de la première extrémité de l'élément ; et
une troisième bande (13) ;
une quatrième bande (13) ;
chacune des bandes comportant un connecteur (30) prévu pour être accouplé avec l'extrémité d'une aiguille (78) ;
**caractérisé en ce que**
la première bande est une première bande inférieure ;
la deuxième bande est une deuxième bande inférieure,
la troisième bande est une troisième bande inférieure raccordée à une première longueur à proximité d'une partie centrale de ladite première longueur ; et
la quatrième bande est une quatrième bande inférieure raccordée à une deuxième longueur à proximité d'une partie centrale de ladite deuxième longueur,
une première aiguille incurvée en trois dimensions (60R) prévue pour s'étendre depuis une incision sur le côté droit d'un patient où un tendon d'adducteur droit du patient pénètre dans une partie droite d'une branche pubienne du patient, latéralement à un bord de ladite branche pubienne, au travers du foramen ischio-pubien droit du patient, vers une incision dans le vagin du patient ;
une deuxième aiguille incurvée en trois dimensions (60L) prévue pour s'étendre depuis une incision sur le côté gauche du patient où un tendon d'adducteur gauche du patient pénètre dans une partie gauche de la branche pubienne du patient, latéralement à un bord de ladite branche pubienne, au travers du foramen ischio-pubien gauche du patient, vers l'incision dans le vagin du patient ;
une troisième aiguille incurvée en trois dimensions (60R) prévue pour s'étendre depuis une incision sur le côté droit du patient où un bord inférieur droit de la branche pubienne du patient aboutit en bas du foramen ischio-pubien droit du patient, au travers du foramen ischio-pubien droit du patient, vers l'incision dans le vagin du patient ; et
une quatrième aiguille incurvée en trois dimensions (60L) prévue pour s'étendre depuis une incision sur le côté gauche du patient où un bord inférieur gauche de la branche pubienne du patient aboutit en bas du foramen ischio-pubien gauche du patient, au travers du foramen ischio-pubien gauche du patient, vers l'incision dans le vagin du patient.

2. Ensemble selon la revendication 1, où le dispositif de support comprend une greffe biologique.

3. Ensemble selon la revendication 2, où la greffe biologique est préalablement fixée à chacune des bandes.

4. Ensemble selon la revendication 1, où l'élément de support est tricoté, et le dispositif de support comprend une greffe biologique fixée sur l'élément de support tricoté.

5. Ensemble selon la revendication 1, où l'élément de support est tricoté avec un premier réglage de barres ; et où les bandes sont raccordées par maillage continu à l'élément de support, et les bandes sont tricotées avec un deuxième réglage de barres.

6. Ensemble selon la revendication 5, où
la première bande est raccordée par maillage continu à la première longueur à proximité de la première extrémité de l'élément ;
la deuxième bande est raccordée par maillage continu à la deuxième longueur à proximité de la première extrémité de l'élément ;
la troisième bande est raccordée par maillage continu à la première longueur à proximité d'une partie centrale de la première longueur ; et
la quatrième bande est raccordée par maillage continu à la deuxième longueur à proximité d'une partie centrale de la deuxième longueur.

7. Ensemble selon la revendication 5, où le premier réglage de barres est :
Barre 1 : 1/0, 2/3, 2/1, 2/3, 1/0, 1/2, 1/0, 1/2 ;
Barre 2 : 1/0, 2/3, 2/3, 1/0 ; et
Barre 3 : 2/3, 1/0, 1/2, 1/0, 2/3, 2/1, 2/3, 2/1.

8. Ensemble selon la revendication 1 ou la revendication 7, où chaque connecteur présente une ouverture prévue pour recevoir l'extrémité d'une aiguille, chaque ouverture de connecteur ayant une forme différente ; et où
chaque aiguille a une extrémité dont la forme est prévue pour ne correspondre qu'à une seule ouverture de connecteur.

9. Ensemble selon la revendication 1, où chaque bande ou connecteur présente un repère d'identification.

10. Ensemble selon la revendication 1, où chaque connecteur a une couleur ; et où chaque aiguille a une couleur correspondant à la couleur d'un connecteur correspondant.

11. Ensemble selon l'une des revendications 1 à 10, où chaque bande comporte un fil de tension.

12. Ensemble selon l'une des revendications 1 à 11, où chaque bande est recouverte d'une gaine.

13. Ensemble selon l'une des revendications 1 à 12, où chaque aiguille est fixée à une poignée et chaque poignée a une longueur, un axe longitudinal sur la longueur, des surfaces principales opposées sur la longueur, des côtés secondaires opposés, et où une section transversale de chaque poignée le long de l'axe longitudinal présente une forme oblongue non circulaire.
